(19) Europäisches Patentamt
European Patent Office
Office européen des brevets

(11) Numéro de publication: 0 449 923 B1

(12) # FASCICULE DE BREVET EUROPEEN

(45) Date de publication du fascicule du brevet: **06.09.95**

(51) Int. Cl.$^6$: **C12N 15/00**, C12N 15/90

(21) Numéro de dépôt: **90900900.3**

(22) Date de dépôt: **22.12.89**

(86) Numéro de dépôt internationale :
**PCT/FR89/00673**

(87) Numéro de publication internationale :
**WO 90/07576 (12.07.90 90/16)**

(54) **PROCEDE DE RECOMBINAISON IN VIVO DE SEOUENCES D'ADN PARTIELLEMENT HOMOLOGUES.**

(30) Priorité: **26.12.88 FR 8817185**

(43) Date de publication de la demande:
**09.10.91 Bulletin 91/41**

(45) Mention de la délivrance du brevet:
**06.09.95 Bulletin 95/36**

(84) Etats contractants désignés:
**AT BE CH DE ES FR GB IT LI LU NL SE**

(56) Documents cités:

**Mol. Gen. Genet., vol. 210, no. 2, 1987 Springer-Verlag, (Berlin, DE), A.M. Gase et al., pp. 369-372**

**Journal of General Microbiology, vol. 129, 1983, SGM, (Colchester, GB), M. Mergeay et al., pp. 321-335**

(73) Titulaire: **SETRATECH**
**31, rue de la Liberté**
**F-75019 Paris (FR)**

(72) Inventeur: **RADMAN, Miroslav, CNRS**
**15, Ouai Anatole France**
**75007 Paris (FR)**
Inventeur: **RAYSSIGUIER, Christiane**
**Les Vignes-de-Bures**
**F-91940 Les Ulis (FR)**

(74) Mandataire: **Warcoin, Jacques et al**
**Cabinet Régimbeau,**
**26, avenue Kléber**
**F-75116 Paris (FR)**

EP 0 449 923 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Chemical Abstracts, vol. 102, no. 23, 10 juin 1985, (Columbus Ohio, US) H. Bagci, p. 344, abrégé 200968h

Chemical Abstracts, vol. 102, no. 5, 4 févr. 1985, (Columbus, Ohio, US), J.P. Abastado et al., p. 141, abrégé 40833e

The Embo Journal, vol. 6, no. 6, 1987, IRL Press Ltd, (Oxford, GB), R. Zell et al., pp. 1809-1815

J. Mol. Biol., vol. 197, no. 4, 1987, Academic Press Ltd, (Londres, GB), S. Shenoy et al., pp. 617-626

Chemical Abstracts, vol. 111, no. 17, 23 oct. 1989, (Columbus, Ohio, US), P. Shen et al., p. 157, abrégé 147639j

PNAS USA, vol. 77, no. 2, févr. 1980, (Washington, US), B.W. Glickman et al., pp. 1063-1067

Nature, vol. 342, no. 6248, 23 nov. 1989, (Londres, GB), C. Rayssiguier et al., pp. 396-401

## Description

La présente invention concerne un procédé de recombinaison in vivo de séquences d'ADN partiellement homologues présentant une proportion importante de mésappariements de bases pouvant aller notamment jusqu'à 30 %.

Il est ainsi possible de recombiner des gènes individuels, au niveau de cellules ou d'organismes d'espèces et genres différents ayant partagé les mêmes ancêtres.

La présente invention concerne également un procédé de production d'organismes recombinés par croisement et recombinaison in vivo d'organismes d'espèces et/ou de genres différents, ainsi qu'un procédé de production in vivo de gènes hybrides et donc des protéines hybrides codées par ceux-ci.

Lors de la synthèse de l'ADN, des erreurs peuvent se produire et conduire à des paires de bases non complémentaires appelées mésappariements. Il existe un processus de correction des erreurs (mésappariements et non appariements des bases) dans le DNA. Ce processus fait intervenir un système enzymatique. Ainsi, chez les bactéries Escherichia Coli et Salmonella typhimurium les erreurs sont très rapidement et précisément détectées par deux enzymes (MutS et MutL) permettant à une troisième enzyme (MutU) de dérouler les deux brins d'ADN et à une quatrième enzyme (MutH) de couper le brin néosynthétisé sur une séquence du DNA (GATC) elle-même méthylée plus tard par une autre enzyme (réf. : 1). Les erreurs les plus fréquentes G:T, A:C et plus ou moins une base, sont les plus efficacement réparées. Les erreurs pas ou mal détectées par les enzymes Mut (G:A, C:T et C:C dans certains endroits) ont une structure particulière "ouvrant" les deux brins.

Jusqu'à maintenant, l'obtention d'espèces hybrides ou de gènes hybrides se heurtait à de nombreux problèmes. Pour ce qui est d'espèces hybrides, les tentatives les plus avancées faites in vitro dans le domaine végétal par fusion de cellules se heurtaient au problème majeur de l'instabilité génétique. Quant à l'obtention de gènes ou enzymes hybrides, elle était possible uniquement in vitro par génie génétique.

Le but de la présente invention est de faire de nouvelles espèces hybrides ou, à fortiori, de nouveaux gènes ou enzymes hybrides par recombinaisons intergénériques et/ou interspécifiques in vivo avec une efficacité et une facilité accrue.

Pour ce faire, et selon une première variante de l'invention, on utilise des mutants défectueux dans le système de réparation des mésappariements de bases de l'ADN ou tout autre mutant qui augmente la recombinaison intergénérique.

On peut citer notamment les souches mut, en particulier mut L, S, H ou U de Escherichia coli et Salmonella typhimurium ou encore les souches hex de Streptococcus pneumoniae (réf. : 2) et pms chez la levure (réf. : 3)

En effet, on a découvert selon l'invention que le mécanisme moléculaire de la spéciation , et donc de l'apparition d'une nouvelle espèce, peut impliquer de façon critique l'activité des enzymes de correction des mésappariements et seulement elles. L'invention consiste en effet à exploiter le rôle d'"antirecombinaison" stimulé par les mésappariements de bases d'ADN qu'ont les enzymes de réparation de ces mésappariements. Ce rôle définit un mécanisme moléculaire de la spéciation, c'est-à-dire de la séparation génétique initiale de nouvelles espèces.

Toutefois, au lieu de l'utilisation des mutants défectueux dans le système enzymatique de réparation des mésappariements de bases de l'ADN, on peut inactiver transitoirement ce système le temps d'obtenir les recombinés voulus, notamment par saturation.

Compte tenu du fait que les mutants de réparation des mésappariements sont génétiquement instables, il peut être intéressant d'utiliser une déficience transitoire nécessaire à la construction génétique désirée avant de revenir à la stabilité génétique normale. Le problème principal en biotechnologie classique est l'instabilité génétique des souches "industrielles". Après la sélection du variant voulu, sa propre stabilité génétique est compromise et il reverse vers le type sauvage au cours de la croissance dans le fermenteur.

La stratégie pour l'inactivation transitoire du système de correction des mésappariements selon l'invention est double :

a) Utilisation d'un mutant de correction conditionnel ($mutS^{ts-1}$) d'Escherichia coli pour toute construction génétique chez E. coli, et

b) Saturation, c'est-à-dire inactivation fonctionnelle du système de correction par l'introduction dans la cellule d'un grand nombre de mésappariements. Cette méthode est applicable à n'importe quel organisme.

La présente invention a donc pour objet un procédé de recombinaison intergénérique in vivo de séquences d'ADN partiellement homologues, présentant une proportion importante de mésappariements de bases pouvant aller jusqu'à 30 %, caractérisé en ce qu'on met en présence lesdites séquences dans des cellules ou un organisme dont le système enzymatique de réparation des mésappariements de bases est

défectueux ou a été inactivé transitoirement par saturation le temps d'obtenir la recombinaison entre lesdites séquences d'ADN, lesdites séquences d'ADN à recombiner provenant de bactéries de genres différents lorsqu'elles sont d'origine prokaryote.

Il convient donc le cas échéant que l'une au moins des enzymes soit inactivée, à savoir une enzyme impliquée dans la reconnaissance ou dans la correction proprement dite des erreurs.

Les séquences d'ADN en cause à recombiner peuvent être chromosomiques ou extra-chromosomiques permettant dans ce dernier cas la recombinaison entre des gènes individuels clonés.

Comme application de ce procédé de recombinaison in vivo, la présente invention a également pour objet un procédé de production in vivo de gènes hybrides et de leur protéines codées, caractérisé en ce qu'on met en présence dans ledit organisme défectueux ou inactivé dans le système enzymatique de réparation des mésappariements de bases, deux dites séquences d'ADN consistant en des gènes partiellement homologues issus de deux organismes différents, et on sélectionne le gène hybride recherché ou sa protéine codée. Il s'agit dans ce cas de production in vivo extra-chromosomique.

On pourra introduire par exemple sur des plasmides les deux gènes partiellement homologues codant pour une même fonction mais ayant une séquence différente et des propriétés enzymatiques du produit différentes puis sélectionner sur boîte de Pétri parmi les milliers de différentes recombinaisons obtenues celles qui pourraient nous intéresser. Ceci correspondrait autrement à un travail immense du génie génétique in vitro.

La présente invention fournit également un procédé de production in vivo de gènes hybrides, et de leur protéines codées dans lequel un premier gène d'un premier organisme est porté par un premier plasmide et un second gène partiellement homologue d'un second organisme sont introduits par transformation dans une bactérie dont le système enzymatique de réparation des mésappariements est défectueux ou dans une bactérie dont le système de réparation des mésappariements est transitoirement inactivé les gènes se recombinent et l'on sélectionne le gène hybride recherché ou sa protéine codée.

En particulier, dans le procédé ci-dessus, la bactérie transformée avec les plasmides portant les gènes à recombiner peut être une souche E. coli ou Salmonella typhimurium défectueuse ou transitoirement inactivée dans le système enzymatique de réparation des mésappariements ou tout autre mutant de telle bactérie qui augmente la recombinaison intergénérique.

Avantageusement, pour toute construction chez E. coli, on utilisera une souche thermosensible pour la fonction de réparation des mésappariements, par exemple la souche E. coli $mutS^{ts-1}$ qui est mutatrice $mutS^{-}$ à 42°C et normale $mut^{+}$ à 32°C. On active la recombinaison hétérospécifique à 42°C, on sélectionne le caractère recherché à 32°C.

L'invention a pour objet en application du procédé de recombinaison in vivo selon l'invention un procédé de production de cellules recombinées par des techniques de transformation ou fusion , caractérisé en ce que :

- on effectue une transformation et une recombinaison in vivo :
  - . à l'aide de l'ADN de cellules d'un organisme d'une première espèee et d'un premier genre,
  - . avec l'ADN chromosomique des cellules d'un organisme d'une deuxième espèce et/ou d'un deuxième genre,

  ces cellules de l'organisme de deuxième espèce ou genre étant défectueuses dans les systèmes enzymatiques de réparation des mésappariements ou ayant ledit système inactivé transitoirement, notamment par saturation, ou bien
- on effectue la recombinaison in vivo des chromosomes de ces deux types de cellules après fusion desdites cellules, celles-ci ayant toutes deux un système enzymatique de réparation des mésappariements défectueux ou ayant ledit système inactivé transitoirement notamment par saturation.

L'invention a également pour objet en application du procédé de recombinaison in vivo selon l'invention, un procédé de production d'organismes recombinés par croisement et recombinaison in vivo d'organismes d'espèces et/ou de genres différents, caractérisé en ce qu'on effectue un croisement et une recombinaison in vivo entre :

- un organisme d'une première espèce et d'un premier genre, et
- un organisme d'une deuxième espèce et/ou d'un deuxième genre,

l'un au moins de ces deux organismes étant défectueux dans le système enzymatique de réparation des mésappariements ou ayant ledit système inactivé transitoirement, notamment par saturation.

Dans le procédé de production de cellules ou d'organismes recombinés selon l'invention, on peut produire des cellules ou des organismes recombinés de bactéries, mais aussi de levures, de plantes ou même d'animaux.

En particulier, l'invention a pour objet un procédé de production de bactéries recombinées par croisement et recombinaison de bactéries de genres différents, caractérisé en ce qu'on effectue une

conjugaison ou une transduction in vivo entre

- des bactéries dites réceptrices d'un premier genre qui sont défectueuses dans les systèmes enzymatiques de réparation des mésappariements de base ou dont les systèmes enzymatiques de réparation des mésappariements de bases sont inactivés transitoirement, notamment par saturation, et
- des bactéries dites donneuses, d'un deuxième genre qui comportent un caractère particulier que l'on souhaite transférer aux cellules réceptrices.

Avantageusement, les bactéries dites réceptrices sont en outre défectives dans les sytèmes enzymatiques de restriction de l'ADN.

L'invention s'est traduite de façon spectaculaire dans le domaine de la conjugaison de bactéries. Les deux genres bactériens Escherichia coli et Salmonella typhimurium qui se sont séparés génétiquement il y a 140 millions d'années (Ochman et Wilson 1987) ne se croisent pas du tout aujourd'hui par recombinaison du DNA.

Les croisements par conjugaison entre Salmonella typhimurium et Escherichia coli sont en effet stériles, c'est-à-dire que la recombinaison est absente ou très faible selon le locus considéré (Baron et al. 1959 ; Einsenstark 1965, Mergeay et Gerits 1983). Toutefois, quand on élimine les fonctions MutL ou MutS par exemple, par mutations, de ces souches on obtient de nouveau, après 140 x $10^6$ années, un croisement par recombinaison très efficace (au moins $10^6$ fois plus élevé que chez les bactéries non mutées). Ainsi le rôle des enzymes de réparation dans la stérilité interspécifique et intergénérique est attesté dans des croisements bactériens.

Dans un mode de réalisation particulier du procédé de production de bactéries recombinées selon l'invention, on croise une souche de E. Coli et une souche de Salmonella typhimurium dont l'une au moins (la réceptrice) est défectueuse ou inactivée, notamment par saturation dans son système enzymatique de réparation des mésappariements de bases.

De préférence, on conjuguera une bactérie donneuse du type Hfr avec une bactérie réceptrice du type $F^-$.

Dans un mode de réalisation particulier, on a réalisé selon l'invention la conjugaison entre une une souche E. Coli donneuse Hfr et une souche Salmonella typhimurium $F^-$ mutante défective pour le système enzymatique de réparation des mésappariements de base du type mutS ou mutL, c'est-à-dire défective pour les protéines MutS et MutL qui interviennent dans la reconnaissance des mésappariements.

Le procédé de production des bactéries recombinées selon l'invention peut conduire à la fabrication de souches nouvelles, par exemple des souches de Salmonella pathogènes atténuées, rendues non toxiques par croisement avec des souches Escherichia coli mais portant toujours les déterminants antigéniques de surface des souches pathogènes, ceci pour produire le vaccin contre la Salmonellose correspondante.

En application du procédé de production d'organismes recombinés selon l'invention, un autre objet de la présente invention est un procédé de production in vivo de gènes hybrides et de leurs protéines codées, à partir de deux gènes partiellement homologues caractérisé en ce qu'on prépare des cellules recombinées à partir de cellules dudit premier organisme qui contiennent un premier gène et les cellules dudit deuxième organisme qui contiennent un second gène partiellement homologue, et on sélectionne le gène hybride recherché ou sa protéine codé.

On peut, en particulier, en utilisant les mutants de réparation des mésappariements de base, croiser des bactéries d'origines différentes et produire ainsi des gènes nouveaux et sélectionner les propriétés recherchées. Il s'agit dans ce cas de gènes chromosomiques.

Comme mentionné précédemment, l'utilisation de mutants en système enzymatique de réparation des mésappariements de base peut être remplacée par la saturation du système enzymatique en introduisant par transfection un hétéroduplex d'ADN riche en mésappariements.

La présente invention a enfin pour objet un procédé de mutagenèse inverse ciblée d'un gène dans un organisme ledit gène comportant une base mutée que l'on veut rétablir telle qu'avant sa mutation, caractérisé en ce qu'on introduit un hétéroduplex comportant un nombre élevé de mésappariements pour inactiver par saturation le système enzymatique de correction des mésappariements de l'organisme, et un oligonucléotide consistant dans la séquence d'ADN rétablie telle qu'avant mutation du gène.

Selon ce procédé, il est possible d'obtenir des changements génétiques ciblés avec des oligonucléotides de synthèse en les introduisant en grande quantité dans des cellules pendant une période d'inactivation fonctionnelle du système de réparation des mésappariements.

D'autres avantages et caractéristiques de la présente invention apparaîtront à la lumière des exemples qui vont suivre.

La figure 1 représente le profil de restriction de trois recombinants intergénériques à partir de croisements de Escherichia coli Hfr et Salmonella typhimurium $F^-$.

**EXEMPLE 1 CONJUGAISON DE SOUCHES E. coli Hfr et Salmonella thyphimurim F⁻**

On a réalisé des conjugaisons entre Hfr sauvages d'E. Coli et $F^-$ de Salmonella Typhimurium défectives dans les systèmes de restriction du DNA, et de surcroît défectives pour les sytèmes de réparation des mésappariements (mutants mutL, mutS, mutH ou MutU).

La souche Hfr de E. Coli a son origine de transfert à 76,5 minutes sur la carte et xylose est le marqueur injecté en premier (78'). Les souches $F^-$ de Salmonella possèdent deux auxotrophies (met A et met E) et une incapacité à utiliser le xylose ($xyl^-$) ; elles sont résistantes à la streptomycine. La souche Hfr de E. Coli est sauvage pour ces marqueurs et sensible à la streptomycine ; elle possède deux autres auxotrophies (leucine et thréonine) ce qui permet une triple contresélection lors des conjugaisons. Les souches Hfr de E. Coli et $F^-$ Salmonella $mut^+$, mutL, mutS, mutH ou mutU défectives pour les systèmes de restriction sont cultivées en milieu liquide riche (LB). Lorsqu'elles sont en phase exponentielle (1 à 5 $10^8$/ml), on mélange 1 ml de Hfr et 1 ml de $F^-$ et on filtre immédiatement le mélange sur filtre millipore. Ce filtre est mis à incuber à 37°C sur une boîte de milieu riche préchauffée. On laisse la conjugaison se faire pendant 40 minutes et le filtre est récupéré et mis dans 1 ml de MgSO4 $10^{-2}$M puis vortexé vigoureusement pendant 1 minute pour resuspendre les bactéries et séparer les conjuguants. Les aliquots sont étalés sur des boîtes de milieu sélectif : milieu minimum 63 (réf. : Miller J.H. 1972, Experiments in Molecular Genetics, Cold Spring Harbor Laboratory Cold Spring Harbor, N.Y.) avec glucose (0,4 %) et sans méthionine pour sélectionner les $met^+$ ou avec xylose pour seul sucre (0,4 %) et avec méthionine (100 µg/ml) pour sélectionner les $xyl^+$. Ces deux milieux sélectifs ne contiennent ni thréonine ni leucine mais contiennent de la streptomycine (25 µg/ml) pour assurer une triple contre sélection des Hfr. Les boîtes sont incubées à 37°C 40 heures, 60 heures et 88 heures et les clones recombinés comptés et étudiés. Une expérience témoin correspondant au croisement homospécifique Hfr Coli x $F^-$ Coli est réalisée dans les mêmes conditions.

De surcroît, les souches $F^-$ de Salmonella ont été rendues mutantes pour recA (la protéine recA est indispensable à toute recombinaison homologue).

Les résultats obtenus pour les souches mutL et mutS pour lesquelles l'effet est le plus important sont décrit dans le tableau 1 ci-dessous. Ce tableau indique la fréquence des réceptrices $F^-$ devenues $xyl^+$ par bactérie Hfr donneuse après soustraction des révertants obtenus avec les souches réceptrices seules (incubation 60 heures). Des résultats équivalents ont été obtenus pour le marqueur met.

Tableau 1

| Conjugaisons | Frequence $xyl^+$/Hfr donneuse |
|---|---|
| Coli Hfr + Coli $F^-$ | 1.3 $10^{-1}$ |
| Coli Hfr + Sal. $mut^+$ $F^-$ | 6.7 $10^{-7}$ |
| Coli Hfr + Sal. mutL $F^-$ | 2.1 $10^{-3}$ |
| Coli Hfr + Sal. mutS $F^-$ | 1.7 $10^{-3}$ |
| Coli Hfr + Sal. $mut^+$ recA $F^-$ | 4.3 $10^{-8}$ |
| Coli Hfr + Sal. mutL recA $F^-$ | 1.8 $10^{-8}$ |
| Coli Hfr + Sal. mutS recA $F^-$ | 6 $10^{-8}$ |

La fréquence de recombinaison intergénériques augmente d'un facteur d'au moins $10^4$ pour mutL et mutS, et légèrement plus faible pour mut H et U par rapport aux conjugaisons faites avec Salmonellas $F^-$ défectives pour la restriction du DNA mais sauvages pour les gènes de réparation des mésappariements ($mut^+$). Par sélection de recombinants sur milieux sélectifs, nous avons pû obtenir ces hybrides entre E. Coli et Salmonella Typhimurium qui, nous le pensons, sont des espèces nouvelles "Eschenella" ou "Salmorichia" avec de nouvelles combinaisons de gènes et des gènes recombinés nouveaux.

Des recombinés prototrophes ($met^+$) et capables de fermenter le sucre xylose ($xyl^+$) obtenus par recombinaison interspécifique peuvent être formés par des mécanismes divers. Le plus simple est le remplacement direct du gène, résultat habituel de la conjugaison recA-dépendante entre E. coli ou entre S. typhimurium (conjugaison intraspécifique). Les deux autres mécanismes possibles conduisent à la formation de diploîdes partiels. Ces types de recombinants diffèrent quant à leur stabilité. On s'attend à ce que les remplacements simples soient stables et les diploîdes partiels instables. En conséquence, on a vérifié la stabilité phénotypique des recombinants de chaque croisement en striant sur des boîtes indicatrices (1 % xylose McConkey medium ref. Miller, J.H. Experiments in molecular genetics (Cold Spring Harbor Laboratory, Cold Spring Harbor, New York, 1972)). Dans l'ensemble, les profits de stabilité des recombinants intergénériques révèlent un groupe hétérogène.

Tableau 2

| Analyse des recombinants xyl$^+$ et metE$^+$ metA$^+$ des croisements de conjugaison E. coli Hfr X S. typhimurium F$^-$ | | | | |
|---|---|---|---|---|
| F$^-$ réceptrice | Marqueurs | | Recombinants xyl$^+$ | |
| | % met$^+$ dans xyl$^+$ | % xyl$^+$ dans met$^+$ | stable : Transposon | |
| | | | instable : perte | |
| S. typhimurium : | | | | |
| mut$^+$ | 7.7 (389) | 5.8 (412) | 33:1 | |
| mutL::Tn10 | 3.6 (412) | 9.4 (412) | 8:27 | 7/8, 0/27 |
| mutS::Tn10 | 8.7 (412) | 6.6 (375) | 1:34 | 0/1, 0/34 |
| mutH::Tn5 | 10.7 (412) | 1.9 (412) | 7:28 | 0/7, 0/28 |
| mutU::Tn5 | 2.4 (412) | 22.0 (412 | 19:16 | 19/19, 3/16 |
| E. coli : | | | | |
| mut$^+$ | 63.1 (412) | 18.9 (412) | 20:0 | |

Pour savoir si les gènes mut$^+$ de Escherichia ont remplacé les gènes mut de Salmonella, on a suivi le devenir des transposons insérés dans les gènes mut de Salmonella : si le gène mut de E. coli fonctionnel a remplacé le gène résidant mut portant Tn, alors le recombinant aura perdu à la fois sa résistance aux antibiotiques et son caractère mutant. Ceci s'est effectivement passé pour 7 des 8 des recombinants xyl$^+$ met$^+$ stables obtenus dans le croisement avec une souche réceptrice S. typhimurium mutL::Tn10 suggérant que, excepté pour un recombinant, les séquences d'Escherichia coli ont remplacé les séquences de Salmonella jusqu'à environ 5 min après le dernier gène sélectionné (metA) dans la région mutL. Dans le croisement avec S. typhimurium mutU::Tn5 comme souche réceptrice, le gène mutU a été remplacé chez 19 des 19 recombinants xyl$^+$ stables. Aucun des recombinants xyl$^+$ met$^+$ des croisements de mutS::Tn10 et mutH::Tn5 n'ont montré une perte de transposon ou un remplacement de gène mut car la conjugaison a été interrompue au bout de 40 min et la région chromosomique des gènes mutS, mutH n'a pas été transférée.

La figure 1 montre la liaison physique entre les séquences de Salmonella et Escherichia coli. L'ADN génomique des deux souches parentales et leurs trois recombinants intergénériques xyl$^+$ met$^+$ stables (respectivement des croisements mutL, mutS et mutH) a été coupé par l'enzyme SpeI et les fragments ont été séparés par électrophorèse sur gel à champ pulsé sur un appareil Beckman Gene Line TAFE. Les deux plus grands fragments d'ADN des S. typhimurium F$^-$ parentales (△) sont absents dans les trois recombinants qui, à la place, ont acquis au moins un fragment non parental que l'on pense contenir une jonction d'ADN inter-espèce. En outre, au moins un grand fragment de restriction d'ADN de E. coli Hfr (∇) est observé dans les recombinants L17 et S12 (L17 est le recombinant qui a été déposé sous le numéro I832).

La souche de S. typhimurium utilisée mutL F$^-$ a été déposée sous la référence SL4213 mutL (n° I831) à la CNCM de l'Institut Pasteur.

Un recombinant intergénérique E. coli Hfr/S. typhimurium F$^-$ mutL a aussi été déposé sous la référence SCL17 (n° I832) à la CNCM de l'Institut pasteur.

## EXEMPLE 2 RECOMBINAISON HETEROSPECIFIOUE DANS L'OPERON HISTIDINE : REMPLACEMENTS DES GENES DE SALMONELLA TYPHIMURIUM PAR CEUX D'ESCHERICHIA COLI

La stratégie expérimentale pour mesurer la fréquence de remplacements des gènes par la recombinaison hétérospécifique entre Salmonella typhimurium et Escherichia coli a été la suivante :

(1) On a utilisé comme souche "réceptrice" d'information génétique S. typhimurium LT2 portant deux gènes défectifs dont on a cherché la récupération fonctionnelle par la recombinaison :

- hisD::IS200 avec une mutation rendant la cellule His$^-$ (incapable de synthétiser l'histidine, même en présence d'un intermédiaire histidinol) et
- proAB47 avec une délétion qui rend la souche incapable de synthétiser la proline (pro$^-$) (ref. Lam, S. and Roth, J.F. (1983) Cell 34 951-960 ; Csonka, L.N. (1981) Mol. Gen. Genet. 182 82-86). HisD:IS200 correspond à l'insertion d'un élément transposable inactivé et ne retourne jamais vers hisD$^+$.

La souche donneuse d'information génétique est soit une souche S. typhimurium avec les opérons

histidine et proline intacts (his+ et pro+), soit une souche de S. typhimurium délétée pour l'opéron his -(his644), mais rendu his+ par l'aquisition d'un épisome d' E. coli, F'150 his+ portant l'opéron his d'E. coli (ref. hartman et al., (1971) Adv. Genet. 16, 1-34 ; Low, K.B. (1973) Bact. Rev. 36, 587-607).

(2) Le transfert génétique de la donatrice dans la réceptrice s'effectue à l'aide d'un bactériophage transducteur p22 Hft int3 (Schnieger. H. (1972) Mol. Gen. Genet 119, 75-88). C'est une méthode classique de transfert des gènes (Miller, I.H. "Experiments in Molecular Genetics", Cold Spring Harbor Laboratory, N.Y. 1972) : on produit un stock de phages en le laissant croître sur la souche donneuse puis on infecte la souche réceptrice avec une multiplicité d'infection par bactérie et on étale les bactéries infectées sur les boîtes dites "sélectives" sur lesquelles ne poussent que les bactéries ayant aquies le caractère génétique de la souche donneuse (par exemple, la gélose M63 milieu minimum (voir Miller ci-dessus) avec la proline mais sans l'histidine et avec l'histidinol (30 µg/ml) pour détecter les bactéries hisD+, ou avec histidine et arginine mais sans proline pour détecter les bactéries pro+). On compte les colonies hisD+ ou pro+ après 2 jours d'incubation à 37 °C.

Une façon d'exprimer les variations dans la recombinaison homéologue (intergénérique) par rapport à la recombinaison homologue (intraspécifique) est de déterminer le rapport des transductants hisD+ et pro+ pour un lysat de P22Hft int3 provenant soit de la donneuse hisD+ d'E.coli, soit de la donneuse hisD+ de S. typhimurium (les deux souches ayant les mêmes proAB de Salmonelle).

Le tableau 3A montre ces deux rapports hisD+/proAB+ (hétérospécifiques et homospécifiques) pour la souche sauvage mut+ et les dérivés mutL, mutS, mutU et mutH de la souche réceptrice. On voit que le rapport hisD+/proAB+ pour hisD hétérospécifique augmente de plus de 100 fois dans les bactéries mutS et mutL tandis que dans mutH augmente un peu moins et mutU très peu. Le rapport n'augmente pas plus que d'un facteur de 4 quand la donneuse est hisD+ et proAB+ de la Salmonella. Ces résultats corroborent les résultats de la conjugaison mais d'une façon qualitative.

Le remplacement des gènes est démontré ici en utilisant comme marqueur génétique de la déficience en hisD un transposon défectif Tn10-dcam portant la résistance au chloramphénicol (10 µg/ml) et ayant détruit le gène hisD en s'insérant dans le gène (Eliott, T. et Roth, J.R. (1988) Mol. Gen. Genet. 213, 332-338). Le remplacement du gène défectif hisD::Tn10d-cam par le gène fonctionnel hisD+ d'E.coli aura deux effets phénotypiques concomittants :

(i) La bactérie réceptrice devient hisD+ (croît en présence histidinol ; phénotype désigné Hol+) et

(ii) Elle perd son transposon Tn10d-cam et devient sensible au chloramphénicol.

Le tableau 3B montre qu'aucun transductant hisD+ n'a retenu la résistance au chloramphénicol, tandis que les transductants pro+ (les gènes proAB sont loin du gène hisD) maintiennent leur résistance au chloramphénicol.

Tableau 3 : croisement par transduction

A. Les alleles mutants favorisent la faculté de séquences *E. coli* à donner la prototrophie histidinol à *S. typhimurium*

| Allele mut de souche réceptrice | Croisements hétérospécifiques $Hol^+$ : E. coli $Pro^+$ : S. typhimurium<br>$Hol^+/Pro^+$ (x $10^4$) | Croisements homospécifiques $Hol^+$ : S. typhimurium $Pro^+$ : S. typhimurium<br>$Hol^+/Pro^+$ |
|---|---|---|
| $mut^+$ | 4 | 320 |
| mutL #1 | 355 | 180 |
| mutL #2 | 147 | 150 |
| mutS #1 | 696 | 130 |
| mutS #2 | 451 | 160 |
| mutU #1 | 20 | 460 |
| mutU #2 | 10 | 520 |
| mutH #1 | 37 | 180 |
| mutH #2 | 170 | 190 |

B. Transduction interspécifique par remplacement allelique

| Allele mut de souche réceptrice | Contenu des plaques | Sélection des plaques | Nombre moyen de colonies |
|---|---|---|---|
| $mut^+$ | Pro, Hol | $Hol^+$ | 5 |
| | Pro, Hol, Cam | $Hol^+$ $Cam^r$ | 0 |
| | His, Arg | $Pro^+$ | 478 |
| | His, Arg, Cam | $Pro^+$ $Cam^r$ | 498 |
| mutS | Pro, Hol | $Hol^+$ | 168 |
| | Pro, Hol, Cam | $Hol^+$ $Cam^r$ | 0 |
| | His, Arg | $Pro^+$ | 1344 |
| | His, Arg, Cam | $Pro^+$ $Cam^r$ | 1110 |

Légende :

$Hol^+$ = croissance en présence d'histidinol

Pro = proline

Arg = arginine

Cam = chloramphénicol

## EXEMPLE 3 INACTIVATION PAR SATURATION TRANSITOIRE DU SYSTEME DE REPARATION DES MESAPARIEMENTS

Chez E. coli, le système de correction des erreurs Mut (H, L, S, U) est limité et peut être saturé par la titration c'est-à-dire l'inactivation fonctionnelle de la protéine MutL.

La souche qui est la plus forte mutatrice connue E. coli mutD5 est défectueuse en lecture de correction exonucléasique associée à la DNA polymérase III, donc elle produit beaucoup d'erreurs de réplication.

On a démontré par la transfection avec un DNA hétéroduplex des phages $\phi$X 174 ou $\lambda$ que, en pleine croissance, en milieu riche, ces bactéries sont déficientes en réparation des mésappariements. Mais si l'on arrête la réplication du DNA bactérien (phase stationnaire ou arrêt spécifique de la réplication par une mutation thermosensible) l'activité réparatrice est récupérée totalement. Cette réparation est bloquée si l'on bloque la synthèse protéique de novo par le chloramphénicol. Donc, le système Mut n'est pas seulement saturé mais "mort" et il faut resynthétiser les enzymes ou l'enzyme de réparation.

Un test de réparation des mésappariements in vivo utilise comme substrat des molécules déterminées construites in vitro par la séparation des brins de l'ADN et la reconstitution de duplexes nouveaux du type hétéroduplexes. En utilisant des gènes mutants déterminés quant à leur séquence, il est possible de construire des molécules avec un seul méasappariement déterminé. Nous avons employé un gène mutant dans le gène cI codant pour le répresseur du bactériophage lambda, la protéine responsable de l'état "dormant" prophagique du bactériophage lambda, qui donne un phénotype "clair" à la plage de lyse par rapport aux plages "troubles" du phage sauvage. Il s'agit de la mutation UV23 correspondant à une mutation G:C qui donne A:T au niveau de la quarante troisième paire de bases du gène cI. Donc en fonction du brin choisi, nous avons construit le DNA du phage lambda avec un brin sauvage et un brin portant la mutation UV23, le duplex est, sinon, normal sur environ 50 000 paires de bases, excepté le mésappariement G:T ou A:C au niveau de la mutation UV23. La préparation des stocks du phage lambda et la séparation des brins dans le gradient de chlorure de cesium en présence des polymères poly (U,G) (uridine et guanosine) a été décrit par M. Meselson et R. Yuan (1968) Nature, 217 : 1110-1114.

Comme le système de correction des mésappariements est dirigé par la méthylation (6-méthyl-adénine) des séquences 5'GATC (Radman et Wagner (1988) Scientific American Août 1988 pour la Science, Octobre 1988), nous avons construit un ADN hétéroduplex portant la méthylation sur un seul brin. L'ADN est introduit en une seule copie dans la bactérie E. coli rendue perméable à l'ADN externe par la méthode du chlorure de calcium (Mandel et Miga (1970) J. Mol. Biol. 53 : 159-162 (les bactéries en croissance exponentielle sont tenues en présence de 0,1 M $CaCl_2$ dans la glace, pendant 2,5 heures au moins)). Les bactéries transfectées sont étalées avec la gélose molle sur les boîtes de Petri, incubées pendant la nuit et la descendance phagique de chaque molécule hétéroduplex dans les centres infectieux, est déterminée par le ré-étalement des phages repiqués de chaque centre infectieux. Trois types de centres infectieux sont possibles : pur trouble ($c^+$), pur clair (c), et mixtes contenant les phages $c^+$ et c. Les centres infectieux mixtes contiennent la descendance d'un ADN hétéroduplex non réparé : les deux brins, un $c^+$, l'autre c, se sont repliqués et ont donné la descendence phagique avant qu'une réparation ait pu éliminer une base mésappariée portant le caractère $c^+$ ou c. Donc, un nombre important de centres infectieux mixtes signifie qu'il y a eu peu de réparation et vice versa. En outre la réparation dirigée produit les centres infectieux purs du type porté par le brin méthylé et il y a donc une incidence dans le rapport $c/c^+$ (voir tableau 4 ci-après)

Cette méthodologie a été décrite dans Proc. Natl. Acad. Sci. USA 82 : 503-505 (1985) par Dohet Wagner et Radman.

Le tableau 4 ci-après montre que dans une souche mutatrice mutD5, défectueuse en fidélité de la synthèse de l'ADN produisant donc beaucoup de mésappariements au cours de la réplication de son ADN, la réparation des mésappariements est déficiente.

Tableau 4 : Analyse génétique de la descendence phagique des molécules hétéroduplexes du bactériophage lambda hémiméthylées dans les diverses souches et conditions de croissance

| Souche d'*E. coli* | condition de la culture | Hétéroduplex : $C^+$—G——(r) $me^-$ / C——T——(l) $me^+$<br>Descendance : $c^+$ | $c/c^+$ | c (%) |
|---|---|---|---|---|
| W 3110 ($mut^+$) | 32°<br>42° | 0<br>1 | 4<br>4 | 96<br>95 |
| $C_{600}$ (mutL) | 32°<br>42 | 1<br>1 | 86<br>92 | 13<br>7 |
| W 3110 dna A (ts) | 32°<br>42° | 0<br>0 | 3<br>3 | 97<br>97 |
| KD 1079 (mut D5) | 32°<br>42 | 0<br>1 | 48<br>49 | 52<br>50 |
| KD 1079 dna A (ts) mut D5 | 32°<br>42° | 1<br>0 | 48<br>10 | 51<br>90 |

En comparant les 3 % de centre infectieux mixtes de la bactérie W 3110 dna Ats avec les 48 et 49 % des centres infectieux mixtes chez la bactérie mutD5, il appparaît bien que la réparation des mésappariements est déficiente chez la mutatrice mutD5. En comparant les 49 % de centres infectieux mixtes avec les 86 à 92 % chez le mutant mutL, il apparaît que le défaut en réparation des mésappariements n'est pas total. L'arrêt d'initiation de la réplication de l'ADN bactérien chez les mutants dna Ats à 42°C pendant 2 heures, n'a pas affecté la réparation des mésappariements chez les souches autres que mutD5, dna Ats où on a pu observer la récupération de la réparation des mésappariements avec 10 % de centre infectieux mixtes. Cette récupération n'a pas eu lieu en présence d'un inhibiteur de la synthèse protéique, soit avec 100 µg/ml de chloramphenicol. Donc, en arrêtant la production des mésappariements par la réplication

erronnée, on ne retrouve la réparation que par la synthèse des protéines. Ceci suggère que la réparation des mésappariements implique un "suicide" enzymatique. La saturation correspondrait alors à une inactivation fonctionnelle d'une ou plusieurs protéines Mut. Dans les expériences décrites ci-après nous montrons que la protéine fonctionnelle manquante est la protéine Mut L.

Préparation de l'hétéroduplex circulaire du phage M13 mp2

L'ADN phagique et l'ADN intracellulaire sont préparés par centrifugation CsCl en présence de bromure d'éthidium comme décrit par Brooks et al. (1988) à partir du phage purifié ou après infection par les phages et incubation pendant 30 minutes. Il s'agit de méthodes standards décrites par Maniatis, Fritsch et Sambrook (1982) Molecular Cloning, Laboratory manual, Cold Spring Harbor Laboratory, New York. L'hétéroduplex est préparé en hybridant le brin d'ADN intracellulaire à double brin (RFI) linéarisé avec l'enzyme Ava II, dénaturé 10 minutes à 70 ° C dans l'eau, puis renaturé en présence de l'ADN simple brin pendant 10 minutes à 60 ° C dans le tampon 2x SSC.

L'ADN simple brin est éliminé par la cellulose-benzoylée-naphtaylée en présence de NaCl 0,1 M. L'ADN hétéroduplex avec un mésappariement G:T est gardé dans le tampon Tris 0,05 M et EDTA 0,001 M à pH 8.

Le mésappariement G:T porte son marquage génétique :

5' — GTT — 3' (-) bleue ($lac^+$) $méthyl^-$

3' — TAA — 5' (+) blanc ($lac^-$) $méthyl^+$

Comme précédemment avec l'ADN du phage lambda, une seule molécule entre dans la cellule rendue perméable par la méthode Hanahan (Maniatis et Coll. 1982), basé sur l'effet du calcium dithiothréitol et diméthylsulfoxyde sur les parois cellulaires. Les bactéries transfectées sont diluées et étalées avec la souche indicatrice CSH 50 (del lac-pro, $ara^-$, $pro^-$, str A) en présence de X-gal et IPTG pour permettre la coloration bleue des plaques phagiques $lac^+$ et non celle des phages $lac^-$ (mutants). Les plages de lyses mixtes (blanc/bleu) montrent l'hétéroduplex non réparé.

Le tableau 5 ci-après montre qu'en phase logarithmique de croissance, la fraction des centres infectieux mixtes est semblable chez le mutant mutL défectif en réparation et le mutant mutD. Mais en phase tardive logarithmique avant la phase stationnaire, la fraction des centres infectieux mixtes chez le mutant mutD est la même que chez le type sauvage $mut^+$ donc, la mutatrice mutD est défective en réparation des mésappariements pendant la croissance rapide mais elle retrouve son activité de réparation lorsque la croissance ralentit.

Tableau 5 : Descendance phagique des molécules hétéroduplexes du bactériophage M13 mp2 (hétéroduplex avec mésappariement G:T) dans les souches mutatrices. Effet de la phase de croissance.

| Souche d'E. coli | nature et pourcentage des centres infectieux | | | | | | | |
|---|---|---|---|---|---|---|---|---|
| | phase log. précoce | | | | phase log. tardive | | | |
| | mixtes | blancs | bleus | total | mixtes | blancs | bleus | total |
| KA 796 ($mut^+$) | 0,5 | 92 | 7,5 | 1120 | 6,9 | 76 | 17,1 | 1290 |
| NR 9163 (mutL) | 50,6 | 7,9 | 41,5 | 860 | 47,8 | 6,6 | 45,6 | 1603 |
| NR 9066 (mutD) | 40,7 | 22 | 37,3 | 1013 | 7,5 | 69,8 | 22,5 | 1949 |

Le tableau 6 ci-après montre que les gènes clonés et surexprimés peuvent récupérer l'activité de réparation des mésappariements dans la souche mutD. Spécifiquement, les gènes mutL et mutH et non les gènes mutS et mutU clonés dans le plasmide pBR325 introduits dans la souche mutD, provoquent la récupération quasi totale de l'activité de réparation. En accord avec cette observation, la fréquence des mutations spontanées décroît dans la mutatrice mutD portant le plasmide pBR325 ($mutL^+$) ou pBR325 ($mutH^+$) et non avec les pBR325 ($mutS^+$) ou pBR325 ($mutU^+$) comme il ressort du tableau 7 ci-après.

Tableau 6

| Descendance phagique des molécules hétéroduplexes (mésappariement G:T) du phage M13 mp2 dans la souche mutatrice mutD5 portant les plasmides avec les gènes mutH, mutL, mutS et mutU | | | | |
|---|---|---|---|---|
| Souches d'E. coli | Nature et pourcentage des centres infectieux | | | |
| | mixtes | blancs | bleus | total |
| NR 9066 mutD5 | | | | |
| - sans pBR | 47,8 | 17,7 | 34,4 | 661 |
| + pBR ($mutH^+$) | 6,0 | 77,6 | 16,4 | 1756 |
| + pBR ($mutL^+$) | 1,3 | 73,3 | 26,2 | 872 |
| + pBR ($mutS^+$) | 49,8 | 14,1 | 36,0 | 1186 |
| + pBR ($mutU^+$) | 39,7 | 18,5 | 41,6 | 867 |
| Segregant ($mutL^-$) | 55,0 | 11,6 | 33,4 | 1161 |
| KA 796 ($mut^+$) | 2,6 | 80,3 | 16,9 | 1778 |
| NR 9163 ($mutL^-$) | 48,2 | 6,2 | 45,5 | 1811 |

Tableau 7

| Fréquences des mutations spontanées dans mutD5 portant les plasmides pBR325 (mutH, mutL, mutS ou mutU) | | |
|---|---|---|
| Souches d'E. coli | Fréquence des mutations (x $10^{-6}$) | |
| | $Rif^R$ | $Nal^R$ |
| NR 9066 mutD5 | | |
| - sans pBR | 215,0 | 60,3 |
| + pBR ($mutH^+$) | 42,4 | 8,7 |
| + pBR ($mutL^+$) | 15,3 | 0,71 |
| + pBR ($mutS^+$) | 149,0 | 37,3 |
| + pBR ($mutU^+$) | 165,0 | 36,4 |
| Segregant $Amp^S$ ($mutL^-$) | 245,0 | 55,1 |
| KA 796 ($mut^+$) | 0,006 | 0,0008 |

Ces expériences présentent la première démonstration expérimentale d'une catastrophe d'erreurs ou d'effet avalanche. Un excès d'erreurs au niveau de la synthèse de l'ADN provoque la saturation (l'inactivation) du système de réparation des mésappariements. Le résultat est un défaut double dans les systèmes de la fidélité de réplication.

En introduisant les plasmides $mutH^+$, $mutL^+$, $mutS^+$ et $mutU^+$ dans E. coli mutD5, on peut démontrer qu'il n'y a pas de perte d'activité de réparation quand la fonction MutL est surproduite. Donc, la protéine MutL se "suicide" dans l'acte de réparation. La mutatrice mutD5 présente le premier cas expérimental de "catastrophe d'erreurs" : trop d'erreurs au niveau de la réplication du DNA qui surcharge le système de correction lequel s'effondre et provoque un effet d'"avalanche".

Donc, il suffit d'introduire d'un coup un nombre excessif de mésappariements de base dans une cellule pour que le système de réparation s'effondre et reste inactif jusqu'à la "dilution" du substrat avec les mésappariements et la resynthèse des enzyme Mut.

**EXEMPLE 4 SATURATION DU SYSTEME DE CORRECTION DES MESAPPARIEMENTS CHEZ LES CELLULES DES MAMMIFERES**

Il est difficile d'isoler un mutant mut- chez les cellules de mammifères. Il s'agit de mutations récessives dans des cellules diploîdes qui nécessitent l'inactivation simultanée de deux copies du même gène. Ces mutants peuvent en outre être létaux. Ceci est dû au fait que le polymorphisme des séquence, surtout au niveau des diverses familles de séquences répétitives, est l'élément clé de la stabilité chromosomique.

C'est en effet grâce à ce polymorphisme que le système de réparation des mésappariements peut empêcher toute recombinaison dangereuse entre les séquence répétitives (aberrations chromosomiques) ou entre les chromosomes homologues (homozygotie par recombinaison mitotique), sauf la recombinaison réparatrice entre les chromatides soeurs (échange de chromatides soeurs) issues de la réplication d'une molécule-mère et ayant donc une séquence identique.

Trois expériences ont été tentées : suite à l'introduction dans les cellules de la souris de DNA hétéroduplex riche en mésappariements, on a cherché à déterminer si l'on pouvait :

a) observer l'apparition d'aberrations chromosomiques provoqués par l'activation de la recombinaison entre les séquences répétitives diversifiées,

b) observer l'apparition de mutations suite à des erreurs de réplication non corrigées,

c) cibler par un oligonucléotide synthétique une mutation cancerogène puis la corriger par un autre oligonucléotide

a) On réalise la transfection des cellules CHO (Chinese Hamster ovary) et NIH3T3 (fibroblastes de la souris) par la méthode décrite par C.Chen et H. Okayama (1987) Mol Cell. Biol. 7 : 2745-2752 ("High efficiency tranformation of mammalian cells by plasmid DNA"). Environ 50 % des cellules sont effectivement transfectées par le DNA circulaire qu'est l'hétéroduplex M13/fd dont la préparation sera explicitée ci-après et environ $10^6$ des paires de bases du DNA entrent dans la cellules, donc environ 200 molécules portant environ 35 000 mésappariements.

Les plaques métaphasiques pour observer les chromosomes mitotiques condensés sont préparées d'après les méthodes classiques décrites par A.R. Kinsella et M. Radman "Inhibition of carcinogen-induced chromosomal aberrations by an anticarcinogenic protease inhibitor", Proc. Natl. Acad. Sci. USA (1980) 77, 3544-3547 et "Tumor promoter induces sister chromatid exchanges : relevance to mechanisms of carcinogenesis", Proc. Natl. Acad. Sci. USA (1978) 75, 6149-6153.

b) Pour tester l'effet mutateur de la transfection par hétéroduplex riche en mésappariements, on mélange le DNA du plasmide pcD neo (Chen et Okayama (1987) Mol. Cell. Biol. 7 : 2745-2752 "high efficiency tranformation of mammalian cells by plasmid DNA") avec l'hétéroduplex. Ceci permet la sélection en présence de 400 µg/ml de néomycine G418 des cellules dans lesquelles le DNA exogèna pénétré. Parmi ces cellules, on teste la fréquence des mutations de résistance à la 6-thioguanine et à l'Ouabaine comme décrit par Kinsella et Radman (1978) P.N.A.S. USA 75, 6149-6153.

c) Mutagenèse ciblée et progammée.

Pour effectuer la pénétration des oligonucléotides de synthèse on peut employer plusieurs méthodes, telles que la méthode du phosphate de calcium, l'électroporation et la méthode des liposomes comme décrite par Chen et Okayama, Andreason et Evans et Mannins et Gould-Fogente respectivement dans Biotechniques, vol 6, n° 7, Juillet/Août 1988). La technique par microinjection directement dans le noyau peut également être pratiquée.

On utilise des cellules NIH3T3 de la souris pour la transformation et l'hétéroduplexe M13/fd dont la préparation est explicitée ci-après comme inactivateur du système de correction des mésappariements ainsi que l'oligonucléotide de synthèse 19-mer 5' GTTGGAGCTTGTGGCGTAG (le T souligné est la base "muté" dans beaucoup de tumeurs humaines et de rongeurs qui se trouve dans le codon 12 de l'oncogène K-ras). Le caractère cancérogène de la cellule transformée se voit par la croissance en "foyer" sur le tapis monocouche ou par la croissance dans la gélose molle. Par mutagenèse reverse avec l'oligonucléotide

5' GTTGGAGCTGGTGGCGTAG, on peut obtenir une guérison génétique.

Préparation de l'hétéroduplexe M13/fd et l'hétéroduplexe $\phi X_1$ 74/G4

On a réalisé un hétéroduplex comportant 3 % de mésappariements entre les DNAs des phages bactériens M13 et fd.

Le principe opératoire est le suivant :

- isoler le DNA intracellulaire (circulaire, double brin ou RFI),
- cliver chaque population moléculaire une fois mais à différents endroits de la molécule,
- dénaturer - renaturer,

- isoler le DNA circulaire (comme décrit par Brooks et al., 1989).

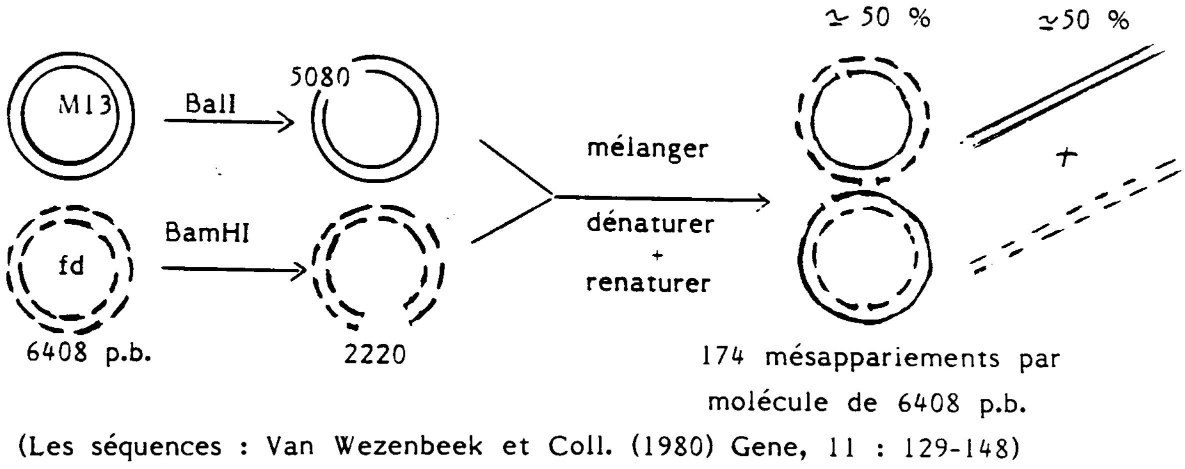


Les cultures de bactéries, infections phagiques, isolement de la forme réplicative (RFI) des bactériophages M13 ou fd ont été décrits par Messing dans Methods in enzymology vol. 101, p 20-78 (1983).

La forme I (RFI) est coupée pour le phage M13 par BalI et pour le phage fd par BamHI. Après digestion enzymatique, les ADN sont dénaturés puis renaturés selon le protocole décrit par Lee, Clark et Modrich PNAS 80 4639-4643 (1983). Après action de la ligase d'E. coli, l'ADN circulaire clos par liaison covalente est purifié par centrifugation en CsCl-EtdBr.

On a également réalisé un hétéroduplex comportant 30 % de mésappariements entre les DNA des phages $\phi$X 174 et G4 selon le même procédé.

Les souches suivantes ont été déposées à la Collection Nationale de Cultures de Microorganismes, Institut Pasteur, 28, rue du Docteur Roux, 75724 PARIS CEDEX 15. en date du 23 DECEMBRE 1988,

Salmonella typhimurium SL4213 mut L N° I 831.

Recombinant Intergénérique SCL17 N° I 832.

REFERENCES BIBLIOGRAPHIQUES

1. MARINUS M. and MORRIS N. (1973) J. Bacteriol 114 1143-1150.
2. TIRABY G. and SICARD A. (1973) J. Bacteriol 116 1130-1135.
3. WILLIAMSON et al. (1965) Genetics 110 609-646.
4. Ochman and Wilson in E. coli and Salmonella typhimurium Vol. 2 American Society for Microbiology Washington DC pp 1649-1654.
5. BARON et al (1959) Proc. Natl. Acad. Sci. USA 45 976-984.
6. EISENSTARK (1965) Proc. natl. Acad. Sci. USA 54 117-120.
7. MERGEAY and GERITS (1983) J. Gen. Microbiol 129 321-335.
8. P. BROOKS et al. accepted for Proc. Natl. Acad. Sci. USA.
9. Maniatis et coll. (1982) Molecules cloning Cold Spring harbor Laboratory.
10. Meselson and yaun (1968) Nature 217 1110-1114.
11. Radman and Wagner (1988) Scientific american August 1988 ("Pour la Science" October 1988).
12. Mendel and Miga (1970) M. Mol. Biol 53 159-162.
13. Dohet and Wagner (1985) Proc. Natl. Acad. Sci. USA 82 503-505.
14. C. Chan, H. Okayama (1987) Mol. Cell. biol. 7 2745-2752.
15. Kinsella and Radman (1978) PNAS 75 6149-6153.
16. Kinsella and Radman (1980) PNAS 77 3544-3547.
17. Biotechniques Vol. 6 n° 7 July/August (1988).
18. Methods in enzymology Vol. 101 pp. 20-78 (1983).
19. PNAS 80 4639-4643 (1983).

**Revendications**

1. Procédé de recombinaison intergénérique in vivo de séquences d'ADN partiellement homologues présentant jusqu'à 30 % de mésappariements de base, caractérisé en ce qu'on met en présence

lesdites séquences dans des cellules ou un organisme dont le système enzymatique de réparation des mésappariements est défectueux ou a été inactivé transitoirement, notamment par saturation, le temps d'obtenir la recombinaison entre lesdites séquences d'ADN, lesdites séquences d'ADN à recombiner provenant de bactéries de genres différents lorsqu'elles sont d'origine prokaryote.

**2.** Procédé de production in vivo de gènes hybrides et de leurs protéines codées, caractérisé en ce que dans un procédé selon la revendication 1 on met en présence dans ledit organisme défecteux ou inactivé dans le système enzymatique de réparation des mésappariements, deux dites séquences d'ADN consistant en des gènes partiellement homologues issus de deux organismes différents, et on sélectionne le gène hybride recherché ou sa protéine codée.

**3.** Procédé de production in vivo de gènes hybrides, et de leur protéines codées selon la revendication 2, dans lequel un premier gène d'un premier organisme est porté par un premier plasmide et un second gène partiellement homologue d'un second organisme sont introduits par transformation dans une bactérie dont le système enzymatique de réparation des mésappariements est défectueux ou dans une bactérie dont le système de réparation des mésappariements est transitoirement inactivé, les gènes se recombinent et l'on sélectionne le gène hybride recherché ou sa protéine codée.

**4.** Procédé selon la revendication 3, caractérisé en ce que la bactérie transformée avec les plasmides portant les gènes à recombiner peut être une souche E. coli ou Salmonella typhimurium défectueuse ou transitoirement inactivée dans le système enzymatique de réparation des mésappariements ou tout autre mutant de telle bactérie qui augmente la recombinaison intergénérique.

**5.** Procédé selon l'une des revendications 1 ou 2, caractérisé en ce qu'on utilise une souche de E. coli $mutS^{ts-1}$ thermosensible pour la fonction de réparation des mésappariements de bases, souche qui est mutatrice $mutS^{-}$ à 42°C et normale $mut^{+}$ à 32°C.

**6.** Procédé de production de cellules recombinées par des techniques de transformation ou fusion selon la revendication 1, caractérisé en ce que :

- on effectue la transformation et la recombinaison in vivo :
  . à l'aide de l'ADN de cellules d'un organisme d'une première espèce et d'un premier genre, et
  . avec l'ADN chromosomique des cellules d'un organisme d'une deuxième espèce et/ou d'un deuxième genre,

  ces cellulles de l'organisme de deuxième espèce ou genre étant défectueuses dans le système enzymatique de réparation des mésappariements de bases ou ayant ledit système inactivé transitoirement, notamment par saturation, ou bien
- on effectue la recombinaison in vivo des chromosomes de ces deux types de cellules après fusion desdites cellules, celles-ci ayant toutes deux un système enzymatique de réparation des mésappariements défectueux ou ayant ledit système inactivé transitoirement notamment par saturation.

**7.** Procédé de production d'organismes recombinés par croisement et recombinaison in vivo d'organismes d'espèces et/ou de genres différents selon la revendication 1, caractérisé en ce qu'on effectue le croisement in vivo entre :

- un organisme d'une première espèce et d'un premier genre, et
- un organisme d'une deuxième espèce et/ou d'un deuxième genre,

l'un au moins de ces deux organismes étant défectueux dans le système enzymatique de réparation des mésappariements de bases ou ayant ledit système inactivé transitoirement, notamment par saturation.

**8.** Procédé selon la revendication 6 ou 7, caractérisé en ce qu'on produit des bactéries, des levures, des cellules de plantes ou animales recombinées à partir d'organismes correspondants.

**9.** Procédé de production de bactéries recombinées par croisement et recombinaison in vivo de bactéries de genres différents selon la revendication 6 ou 7, caractérisé en ce qu'on effectue une conjugaison ou transduction in vivo entre

- des bactéries dites réceptrices d'un organisme d'un premier genre qui sont défectueuses dans les systèmes enzymatiques de réparation des mésappariements de base ou dont les systèmes

enzymatiques de réparation des mésappariements des bases sont inactivés transitoirement notamment par saturation le temps d'obtenir la recombinaison voulue, et

- des bactéries dites donneuses d'un organisme d'un deuxième genre qui comporte un caractère particulier que l'on souhaite transférer aux bactéries réceptrices.

**10.** Procédé selon la revendication 9, caractérisé en ce que les bactéries dites réceptrices sont en outre défectives dans le système enzymatique de restriction de l'ADN.

**11.** Procédé de production in vivo de gènes hybrides et de leurs protéines codées à partir de deux gènes partiellement homologues, caractérisé en ce que dans un procédé selon l'une des revendications 7 à 10 un premier organisme contient un premier gène et ledit deuxième organisme contient le second gène partiellement homologue, et en ce qu'on sélectionne le gène hybride recherché ou sa protéine codée.

**12.** Procédé selon l'une des revendications 9 à 11, caractérisé en ce qu'on croise une souche E. Coli et une souche Salmonella typhimurium dont l'une au moins est défectueuse ou inactivée transitoirement dans le système enzymatique de réparation des mésappariements.

**13.** Procédé selon la revendication 12, caractérisé en ce que la souche E. coli ou Salmonella typhimurium défectueuse dans le système enzymatique de réparation des mésappariements de base est une souche $mutS^-$ ou $mutL^-$, c'est-à-dire défectueuse en protéine MutS ou MutL qui interviennent dans ta reconnaissance des mésappariements.

**14.** Procédé selon l'une des revendications 9 à 13, caractérisé en ce que la bactérie donneuse est une souche HFr, la bactérie réceptrice est une souche mutante $F^-$, en cas de conjugaison.

**15.** Procédé selon l'une des revendications précédentes, caractérisé en ce que la saturation du système enzymatique de réparation des mésappariements de base se fait en introduisant un hétéroduplex riche en mésappariement.

**16.** Procédé de mutagenèse inverse ciblée d'un gène dans un organisme selon la revendication 1, ledit gène comportant une base mutée que l'on veut rétablir telle qu'avant sa mutation, caractérisé en ce qu'on introduit dans l'organisme un hétéroduplex comportant un nombre élevé de mésappariement pour inactiver par saturation le système enzymatique de correction des mésappariements de l'organisme et un oligonucléotide consistant dans la séquence d'ADN rétablie telle qu'avant mutation du gène.

**17.** Procédé selon les revendications 15 ou 16, caractérisé en ce que l'hétéroduplex provient des phages M13 et fd.

## Claims

**1.** Process of intergeneric recombination in vivo of partially homologous DNA sequences having up to 30 % of base mismatches, characterized in that said sequences are placed together in cells or an organism of which the enzymatic mismatch repair system is defective or has been inactivated transitorily, particularly by saturation, for the time to obtain recombination between said DNA sequences, said DNA sequences to recombine, when of prokaryotic origin, coming from bacteria of different genera.

**2.** Process for the in vivo production of hybrid genes and of their coded proteins, characterized in a process according to claim 1, comprising placing together in said organism defective or inactivated in the enzymatic mismatch repair system, two said DNA sequences consisting of partially homologous genes derived from two different organisms, and selecting the desired hybrid gene or its coded protein.

**3.** Process for the in vivo production of hybrid genes, and of their coded proteins according to claim 2, wherein a first gene from a first organism carried on a first plasmid and a second partially homologous gene from a second organism are introduced by transformation in a bacterium of which the enzymatic mismatch repair system is defective or in a bacterium of which the mismatch repair system is transitorily inactivated, and allowed to recombine and wherein the desired hybrid gene or its coded

protein is selected.

**4.** Process according to claim 3, characterized in that the bacteria transformed with the plasmids carrying the genes to recombine may be an E. coli or Salmonella typhimurium strain defective or transitorily inactivated in the enzymatic mismatch repair system or any other mutant of such bacteria which increases the intergeneric recombination.

**5.** Process according to one of the claims 1 or 2, characterized in that a thermosensitive strain of E. coli $mutS^{ts-1}$ is used for the mismatch repair function, which strain is $mut^{S-}$ mutator at 42 °C and normal $mut^{+}$ at 32 °C.

**6.** Process for the production of cells recombined by the transformation or fusion techniques according to claim 1, characterized in that :

- the transformation and the recombination in vivo are performed :
  - by means of the DNA of cells of an organism of a first species and of a first genus, and
  - with chromosomal DNA from the cells of an organism of a second species and/or of a second genus,

  these cells of the organism of the second species or genus being defective in the enzymatic mismatch repair system or having said system transitorily inactivated, particularly by saturation, or
- by performing the in vivo recombination of the chromosomes of these two types of cells after fusion of said cells, the latter having both a defective enzymatic mismatch repair system or having said system transitorily inactivated, particularly by saturation.

**7.** Process for the production of organisms recombined by cross and recombination in vivo of organisms of different species and/or of different genera according to claim 1, characterized in that the in vivo cross is performed between:

- an organism of a first species and of a first genus, and
- an organism of a second species and/or of a second genus,

one at least of these organisms being defective in the enzymatic mismatch repair system or having said system transitorily inactivated, particularly by saturation.

**8.** Process according to claim 6 or 7, characterized in that recombined bacteria, yeasts, plant or animal cells are produced from corresponding organisms.

**9.** Process for the production of bacteria recombined by cross and recombination in vivo of bacteria of different genera according to claim 6 or 7, characterized in that in vivo conjugation or transduction is carried out between :

- so-called recipient bacteria of an organism of a first genus which are defective in the enzymatic mismatch repair systems or of which the enzymatic mismatch repair systems are transitorily inactivated, particularly by saturation, for the time to obtain the desired recombination, and
- so-called donor bacteria of an organism of a second genus which comprises a particular character which it is desired to transfer to the recipient bacteria.

**10.** Process according to claim 9, characterized in that the so-called recipient bacteria are in addition defective in the enzymatic DNA restriction system.

**11.** Process for the production in vivo of hybrid genes and of their coded proteins from two partially homologous genes, characterized in that in a process according to one of the claims 7 through 10, a first organism contains a first gene and said second organism contains the second partially homologous gene, and wherein the desired hybrid gene or its coded protein is selected.

**12.** Process according to one of the claims 9 through 11, characterized in that a cross is performed between an E. coli strain and a Salmonella typhimurium strain of which one at least is defective or transitorily inactivated in the enzymatic mismatch repair system.

**13.** Process according to claim 12, characterized in that the E. coli strain or Salmonella typhimurium defective in the enzymatic mismatch repair system is a $mutS^{-}$ or $mutL^{-}$ strain, that is to say, defective

in MutS or MutL protein which take part in the recognition of the mismatches.

14. Process according to one of the claims 9 through 13, characterized in that the donor bacterium is an HFr strain, the recipient bacterium is an F- mutant strain, in the case of conjugation.

15. Process according to one of the preceding claims, characterized in that the saturation of the enzymatic mismatch repair system is effected by introducing a heteroduplex rich in mismatches.

16. Process of targeted reverse mutagenesis of a gene in an organism according to claim 1, said gene comprising a mutated base that it is desired to reestablish as it was before its mutation, characterized in that into the organism is introduced a heteroduplex comprising a high number of mismatches to inactivate by saturation the enzymatic system of mismatch correction of the organism and an oligonucleotide consisting in the DNA sequence reestablished as before the mutation of the gene.

17. Process according to claims 15 or 16, characterized in that the heteroduplex comes from phages M13 and fd.

**Patentansprüche**

1. Verfahren zur in vivo-Intergenera-Rekombination von partiell homologen DNA-Sequenzen, die bis zu 30 % Basen-Fehlpaarungen aufweisen, dadurch gekennzeichnet, daß man die genannten Sequenzen in Zellen oder in einem Organismus, deren (dessen) enzymatisches System zur Reparatur von Fehlpaarungen defekt ist oder insbesondere durch Sättigung vorübergehend inaktiviert worden ist, zusammenbringt für eine Zeit zur Erzeugung einer Rekombination zwischen den genannten DNA-Sequenzen, wobei die genannten, miteinander zu rekombinierenden DNA-Sequenzen aus Bakterien verschiedener Genera stammen, wenn sie prokaryontischen Ursprungs sind.

2. Verfahren zur in vivo-Bildung von Hybrid-Genen und ihren codierten Proteinen, dadurch gekennzeichnet, daß man in einem Verfahren nach Anspruch 1 in dem genannten Organismus, dessen enzymatisches System zur Reparatur von Fehlpaarungen defekt oder inaktiviert ist, zwei der genannten DNA-Sequenzen, die aus partiell homologen Genen bestehen und aus zwei verschiedenen Organismen stammen, zusammenbringt und das gesuchte Hybrid-Gen oder sein codiertes Protein selektioniert.

3. Verfahren zur in vivo-Bildung von Hybrid-Genen und ihren codierten Proteinen nach Anspruch 2, bei dem ein erstes Gen aus einem ersten Organismus, das von einem ersten Plasmid getragen wird, und ein partiell homologes zweites Gen eines zweiten Organismus durch Transformation in ein Bakterium, dessen enzymatisches System zur Reparatur von Fehlpaarungen defekt ist, oder in ein Bakterium, dessen System zur Reparatur von Fehlpaarungen vorübergehend inaktiviert ist, eingeführt werden, die Gene rekombiniert werden und das gesuchte Hybrid-Gen oder sein codiertes Protein selektioniert wird.

4. Verfahren nach Anspruch 3, dadurch gekennzeichnet, daß das mit den Plasmiden, welche die zu rekombinierenden Gene tragen, transformierte Bakterium ein solches vom Stamm E. coli oder Salmonella typhimurium, das defekt oder vorübergehend inaktiviert ist in bezug auf das enzymatische System zur Reparatur der Fehlpaarungen, oder irgendeine andere Mutante dieses Bakteriums sein kann, welche(s) die Intergenera-Rekombination verbessert.

5. Verfahren nach einem der Ansprüche 1 oder 2, dadurch gekennzeichnet, daß man für die Funktion der Reparatur der Basen-Fehlpaarungen einen wärmeempfindlichen E. coli-Stamm $mutS^{ts-1}$ verwendet, der bei 32 °C zur normalen Mutante $mut^+$ und bei 42 °C zur Mutante $mutS^-$ mutiert.

6. Verfahren zur Bildung von rekombinierten Zellen durch Transformations- oder Fusions-Methoden nach Anspruch 1, dadurch gekennzeichnet, daß man:
   - die Transformation und die Rekombination in vivo durchführt:
     . mit Hilfe der DNA von Zellen eines Organismus einer ersten Species und eines ersten Genus und
     . mit Hilfe der Chromosomen-DNA der Zellen eines Organismus einer zweiten Species und/oder eines zweiten Genus,

   wobei diese Zellen des Organismus der zweiten Species oder des zweiten Genus defekt sind in

bezug auf das enzymatische System zur Reparatur von Basen-Fehlpaarungen oder ein insbesondere durch Sättigung vorübergehend inaktiviertes derartiges System aufweisen, oder
- die in vivo-Rekombination der Chromosomen dieser beiden Zell-Typen nach der Fusion der genannten Zellen durchführt, wobei diese Zellen beide ein defektes oder ein insbesondere durch Sättigung vorübergehend inaktiviertes enzymatisches System zur Reparatur von Fehlpaarungen aufweisen.

**7.** Verfahren zur Bildung von rekombinierten Organismen durch in vivo-Kreuzung und -Rekombination von Organismen verschiedener Species und/oder Genera nach Anspruch 1, dadurch gekennzeichnet, daß man
die Kreuzung in vivo durchführt zwischen:
- einem Organismus einer ersten Species und eines ersten Genus und
- einem Organismus einer zweiten Species und/oder eines zweiten Genus,

wobei mindestens einer dieser beiden Organismen defekt ist in bezug auf das enzymatische System zur Reparatur von Basen-Fehlpaarungen oder ein solches System aufweist, das insbesondere durch Sättigung vorübergehend inaktiviert ist.

**8.** Verfahren nach Anspruch 6 oder 7, dadurch gekennzeichnet, daß man, ausgehend von entsprechenden Organismen, rekombinierte Bakterien, Hefen, Pflanzenzellen oder Tierzellen herstellt.

**9.** Verfahren zur Herstellung von rekombinierten Bakterien durch Kreuzen und Rekombinieren in vivo von Bakterien verschiedener Genera nach Anspruch 6 oder 7, dadurch gekennzeichnet, daß man
eine in vivo-Konjugation oder -Transduktion durchführt zwischen
- sogenannten Empfänger-Bakterien eines Organismus eines ersten Genus, die defekt sind in bezug auf die enzymatischen Systeme zur Reparatur von Basen-Fehlpaarungen oder deren enzymatische Systeme zur Reparatur von Basen-Fehlpaarungen, insbesondere durch Sättigung vorübergehend inaktiviert sind für eine Zeit zur Erzielung der gewünschten Rekombination, und
- sogenannten Donor-Bakterien eines Organismus eines zweiten Genus, der eine spezielle Eigenschaft aufweist, die man auf die Empfänger-Bakterien übertragen will.

**10.** Verfahren nach Anspruch 9, dadurch gekennzeichnet, daß die genannten Empfänger-Bakterien außerdem defekt sind in bezug auf das enzymatische System zur Restriktion der DNA.

**11.** Verfahren zur in vivo-Bildung von Hybrid-Genen und ihren codierten Proteinen, wobei man von zwei partiell homologen Genen ausgeht, dadurch gekennzeichnet, daß in einem Verfahren nach einem der Ansprüche 7 bis 10 ein erster Organismus ein erstes Gen enthält und der genannte zweite Organismus das partiell homologe zweite Gen enthält, und daß man das gesuchte Hybrid-Gen oder sein codiertes Protein selektioniert.

**12.** Verfahren nach einem der Ansprüche 9 bis 11, dadurch gekennzeichnet, daß man einen Stamm E. coli und einen Stamm Salmonella typhimurium, von denen mindestens einer defekt oder vorübergehend inaktiviert ist in bezug auf das enzymatische System zur Reparatur von Fehlpaarungen, miteinander kreuzt.

**13.** Verfahren nach Anspruch 12, dadurch gekennzeichnet, daß der Stamm E. coli oder Salmonella typhimurium, der defekt ist in bezug auf das enzymatische System zur Reparatur von Basen-Fehlpaarungen, ein Stamm mutS$^-$ oder mutL$^-$ist, d.h. defekt ist an dem Protein MutS oder MutL, die an der Erkennung der Fehlpaarungen beteiligt sind.

**14.** Verfahren nach einem der Ansprüche 9 bis 13, dadurch gekennzeichnet, daß im Fall der Konjugation das Donor-Bakterium ein Stamm HFr und das Rezeptor-Bakterium ein Mutanten-Stamm F$^-$ ist.

**15.** Verfahren nach einem der vorhergehenden Ansprüche, dadurch gekennzeichnet, daß die Sättigung des enzymatischen Systems zur Reparatur von Basen-Fehlpaarungen durch Einführen eines an Fehlpaarungen reichen Heteroduplex erfolgt.

**16.** Verfahren zur gezielten inversen Mutagenese eines Gens in einem Organismus nach Anspruch 1, wobei das genannte Gen eine mutierte Base aufweist, die man wieder in ihren Zustand vor ihrer

Mutation überführen will, dadurch gekennzeichnet, daß man in den Organismus ein Heteroduplex einführt, das eine erhöhte Anzahl von Fehlpaarungen aufweist, um das enzymatische System des Organismus zur Korrektur von Fehlpaarungen durch Sättigung zu inaktivieren, und ein Oligonucleotid einführt, das besteht aus der DNA-Sequenz die wieder in den Zustand vor der Mutation des Gens überführt worden ist.

**17.** Verfahren nach Anspruch 15 oder 16, dadurch gekennzeichnet, daß das Heteroduplex aus den Phagen M13 und fd stammt.

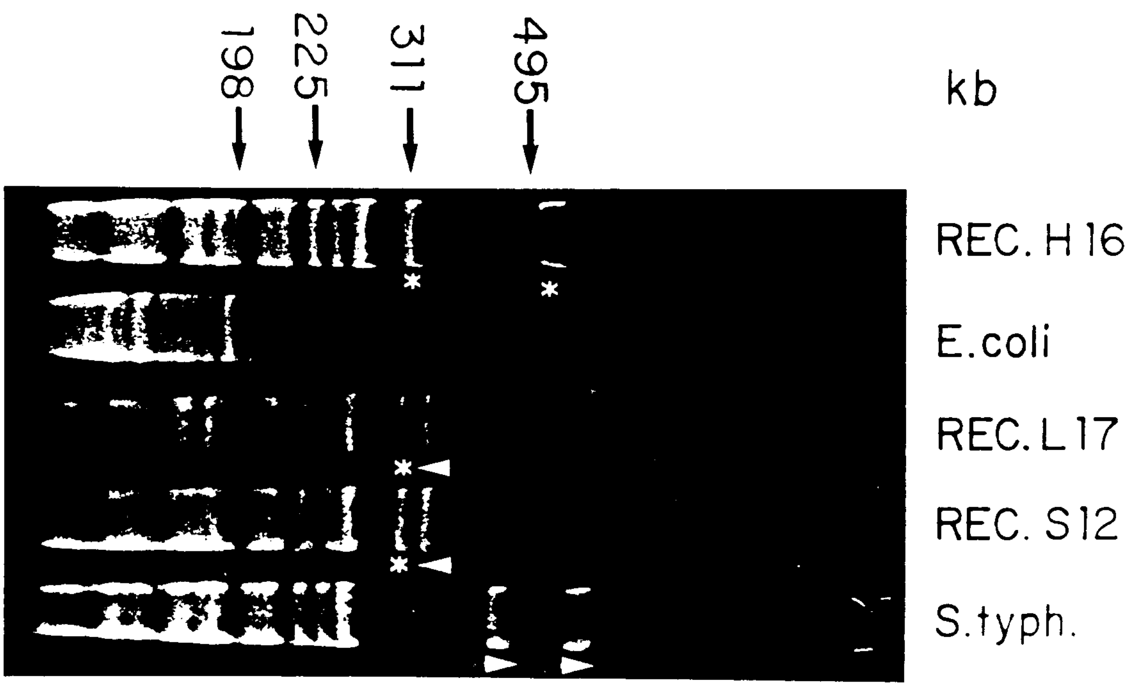
198
225
311
495
kb
REC. H 16
E.coli
REC. L 17
REC. S 12
S.typh.